Europäisches Patentamt

European Patent Office

Office européen des brevets

(11) Veröffentlichungsnummer: **0 596 908 B1**

(12) **EUROPÄISCHE PATENTSCHRIFT**

(45) Veröffentlichungstag der Patentschrift: **11.10.95**

(51) Int. Cl.⁶: **A61K 7/20**, A61K 7/16

(21) Anmeldenummer: **92914597.7**

(22) Anmeldetag: **09.07.92**

(86) Internationale Anmeldenummer:
**PCT/EP92/01558**

(87) Internationale Veröffentlichungsnummer:
**WO 93/00884 (21.01.93 93/03)**

(54) **DURCH SILBERKOLLOID STABILISIERTES WASSERSTOFFPEROXIDENTHALTENDES MUNDHYGIENEMITTEL.**

(30) Priorität: **13.07.91 DE 4123292**

(43) Veröffentlichungstag der Anmeldung:
**18.05.94 Patentblatt 94/20**

(45) Bekanntmachung des Hinweises auf die Patenterteilung:
**11.10.95 Patentblatt 95/41**

(84) Benannte Vertragsstaaten:
**AT BE CH DE DK ES FR GB GR IT LI LU MC NL SE**

(56) Entgegenhaltungen:
**EP-A- 0 390 456**
**AT-A- 349 640**
**DE-A- 1 767 605**
**FR-A- 764 670**
**GB-A- 2 189 394**

(73) Patentinhaber: **HUNGERBACH, Ulrike**
**Windorfer Strasse 295**
**D-56290 Mörsdorf (DE)**

(72) Erfinder: **HUNGERBACH, Heinz**
**Gartenstrasse 4**
**D-56290 Heyweiler/Beltheim (DE)**
Erfinder: **STRUZINA, Werner**
**Gladiolenweg 2**
**D-5628 Heiligenhaus (DE)**
Erfinder: **HOBURG, Albrecht**
**Schumannsdieken 16**
**D-4030 Ratingen 4 (DE)**

(74) Vertreter: **Werner, Hans-Karsten, Dr. et al**
**Patentanwälte**
**von Kreisler-Selting-Werner,**
**Deichmannhaus (Bahnhofsvorplatz)**
**D-50667 Köln (DE)**

Rank Xerox (UK) Business Services
(3.10/3.09/3.3.3)

**Beschreibung**

Gegenstand der Erfindung sind Mundhygienemittel, ein Verfahren zu ihrer Herstellung und eine Verwendung.

Wasserstoffperoxid wurde bisher als Bleich- und Reinigungsmittel verwendet. Es ist bekannt, Wasserstoffperoxid zur Entgiftung, Desodorierung von Wasser, Abwasser und Abluft anstelle von Chlordioxid zu verwenden. Eine 3%-ige Wasserstoffperoxid-Lösung ist für die Munddesinfektion geeignet.

Die Anwendung von Wasserstoffperoxid, insbesondere im Desinfektionsbereich, gewinnt zunehmend Bedeutung, da diese Substanz erheblich umweltfreundlicher ist verglichen mit den bisher weitgehend verwendeten chlorhaltigen Desinfektionsmitteln.

Die Gebrauchsfähigkeit und Lagerfähigkeit von wasserstoffperoxidhaltigen Lösungen ist jedoch dadurch begrenzt, daß Wasserstoffperoxid besonders in der Wärme, im Licht, in Gegenwart von Staub, Schwermetallspuren und alkalisch reagierenden Substanzen in Wasser mehr oder weniger schnell in Sauerstoff zerfällt. Bei der Desinfektion oder Bleichung ist dies ein durchaus erwünschtes Phänomen, da der beim Zerfall entstehende Sauerstoff desinfizierend und bleichend wirkt. Bei der Lagerung solcher Lösungen ist dies jedoch von Nachteil, da sich die wasserstoffperoxidhaltigen Lösungen nach einiger Zeit zersetzen und ihre desinfizierende Wirkung verlieren.

Aus der EP-A-0 325 267 sind Peroxid-Gele bekannt, die 35 bis 95 Gew.-% Polyole enthalten und zusätzlich einen Stabilisator und Wasserstoffperoxid. Als Stabilisator werden Antioxidantien oder U.V. absorbierende Materialien genannt, wie butoxiliertes Hydroxyanisol, Hydroxytoluol, Propylgallat oder andere substituierte Benzophenone. Obwohl es heißt, daß diese toxikologisch akzeptabel sein sollen, sind sie zum Teil nicht unproblematisch.

Aus der AT-PS 349 640 ist eine poliermittelfreie Zahnpasta bekannt, die im wesentlichen aus einer Öl-in-Wasser-Emulsion besteht. Die Stabilisierung des Wasserstoffperoxids erfolgt mit Harnstoff.

Aus der EP-A-0 288 420 ist ein wäßriges Wasserstoffperoxidgel bekannt, welches mit Polyoxyethylen-polyoxypropylen-Block-Copolymeren geliert ist und Polyethylenglycol zur Feuchthaltung enthält. Die darin verwerteten Substanzen, insbesondere Pluronics, sind toxikologisch nicht unbedenklich.

Es war daher das technische Problem der Erfindung, ein stabilisiertes Wasserstoffperoxid für Mundhygienemittel zur Verfügung zu stellen, welches einerseits die Herstellung gebrauchsfähiger und lagerbeständiger Mundhygienemittel ermöglicht und andererseits über die bereits bekannten vorteilhaften desinfizierenden Wirkungen des Wasserstoffperoxids verfügt.

Dieses technische Problem wird durch ein Mundhygienemittel gelöst, das eine mit einem Silberkolloid stabilisierte Wasserstoffperoxid-Lösung in einer Konzentration von 0,1 bis 10 Gew.-%, vorzugsweise 1 bis 3 Gew.-% neben üblichen Inhaltsstoffen enthält. Eine solche stabilisierte Wasserstoffperoxid-Lösung ist beispielsweise von der Firma Hungerbach-Chemotechnik GmbH unter der Bezeichnung HUWA-SAN erhältlich. Diese Lösung ist mit einem Silberkolloid stabilisiert.

Das erfindungsgemäße Mundhygienemittel wird durch Mischen der üblichen Inhaltsstoffe mit der stabilisierten Wasserstoffperoxidlösung hergestellt.

Das technische Problem der Erfindung wird weiterhin gelöst durch die Verwendung einer mit einem Silberkolloid stabilisierten Wasserstoffperoxidlösung für die Mundhygiene in einer Konzentration von 0,1 bis 10 Gew.-%, vorzugsweise 1 bis 3 Gew.-%.

Die mit einem Silberkolloid stabilisierte Wasserstoffperoxid-Lösung kann in einer Vielzahl von Mundhygienemitteln zur Anwendung kommen. Dazu gehören Zahnpasta und Zahnpasta für empfindliche Zähne, die beide im allgemeinen lösliche Kalium- und Strontiumsalze oder Hydroxylapatit enthalten, sowie Mundwasser, Zahnpulver, Kaugummi zur Zahnpflege. Weiterhin ist eine Anwendung in Mundhygienemitteln für dritte Zähne, wie Haftmitteln, Haftschaum, Reinigungsmitteln für dritte Zähne, möglich. Die stabilisierte Wasserstoffperoxid-Lösung kann auch als Konzentrat oder fertige Lösung zum Befüllen von Mundduschen verwendet werden.

Mit der mit einem Silberkolloid stabilisierten Wasserstoffperoxid-Lösung werden trotz der Stabilisierung hervorragende desinfizierende Wirkungen erreicht. So wurde eine Untersuchung der desinfizierenden Wirkung einer stabilisierten $H_2O_2$-Lösung (HUWA-SAN) durchgeführt. Hierbei wurde festgestellt, daß eine wirksame Desinfektion in wasserführenden Systemen im Krankenhaus durch stabilisiertes Wasserstoffperoxid erreicht werden kann.

Weiterhin wurde eine Desinfektionsprüfung durchgeführt mit verschiedenen Keimen und verschiedenen Konzentrationen von stabilisiertem Wasserstoffperoxid (HUWA-SAN). Wie aus Tabelle 1 ersichtlich ist, zeigte sich bis zu einer Konzentration von 0,0075 % bei allen Stämmen kein Wachstum. Die stabilisierte Wasserstoffperoxid-Lösung besitzt aufgrund dieser Ergebnisse eine deutliche bakteriostatische und fungistatische Wirkung. Die minimale Hemmkonzentration liegt bei 5 ppm. Im Mittel kann mit einem Wert für alle Keime von 30 ppm gerechnet werden.

Tabelle 1

Konzentrationsabhängige Keimhemmung
durch stabilisierte Wasserstoffperoxid-Lösung (HUWA-SAN)

| Testkeim | Eingesetzter Keimgehalt/ml | Prüfkonzentration in % | | | | | | | | | |
|---|---|---|---|---|---|---|---|---|---|---|---|
| | | 10 | 1 | 0,1 | 0,05 | 0,01 | 0,0075 | 0,005 | 0,002 | 0,001 | 0,0005 |
| Escherichia coli | $53 \times 10^6$ | - | - | - | - | - | - | - | - | - | (+) |
| Klebsiella pneumoniae | $6,1 \times 10^6$ | - | - | - | - | - | - | - | - | - | + |
| Staphylo- coccus | $50 \times 10^6$ | - | - | - | - | - | - | - | - | - | - |
| Pseudomonas aeruginosa | $70 \times 10^6$ | - | - | - | - | - | - | - | + | ++ | ++ |
| Streptococcus faecalis | $36 \times 10^6$ | - | - | - | - | - | - | - | - | - | (+) |
| Proteus mirabilis | $24 \times 10^6$ | - | - | - | - | - | - | - | - | - | - |
| Mycobacterium tuberculosis | $1,5 \times 10^6$ | - | - | - | - | - | - | (+) | + | + | + |
| Clostridium sporogenes | $2,1 \times 10^6$ | - | - | - | - | - | - | - | + | ++ | ++ |
| Candida albicans | $3,2 \times 10^6$ | - | - | - | - | - | - | (+) | ++ | ++ | ++ |

Zeichenerklärung:

- = kein Wachstum
(+) = schwaches Wachstum
+ = deutliches Wachstum
++ = starkes Wachstum
BLW = Blindwert mit sterilem Wasser

In einer weiteren Untersuchung wurde die Desinfektionswirkung in Mundhygienemitteln mit einer 2%-igen stabilisierten Wasserstoffperoxid-Lösung (HUWA-SAN) ermittelt. Dazu wurden die Keime Pseudomonas aeruginosa, Candida albicans und Staphylococcus aureus eingesetzt. Zur Testung wurden Kunststoffplättchen aus Prothesenkunststoff in der Größe 2,5 cm x 2,5 cm x 0,3 cm gefertigt. Die Testkörper wurden mit folgender Kontaminationslösung mikrobiell kontaminiert:

10 ml 0,9 % sterile Natriumchlorid-Lösung mit dem Zentrifugat einer 200 ml Anzucht einer der Kulturen,

83 ml 5 % sterile Muzin Lösung,
2 ml 5 % sterile Calciumchlorid-Lösung.

Die Testkörper wurden für 30 Sekunden in die Kontaminationslösung gelegt. Die Entnahme erfolgte mit einer Pinzette und anschließender Ablage auf einem feinmaschigen Gitter. Dort erfolgte eine Antrocknung für 15 Minuten bei Zimmertemperatur. Die kontaminierten Testkörper wurden allseitig eingeschäumt mit einem Haftschaum, der mit einem sterilen Glasspatel verteilt wurde. Nach Ablauf der Einwirkzeiten wurden die Testkörper mit einer Pinzette auf ein steriles Baumwolltuch gelegt und vom anhaftenden Schaum befreit. Die Kontrollen wurden ebenfalls abgetrocknet.

Die Keimzahlbestimmung wurde per Ausschüttelung in folgender Ausschüttellösung ermittelt:

Substanzen in 100 ml CSL
3 % Tween 80
3 % Saponin
0,1 % Histidin
0,1 % Cystein.

Nach einer Schüttelzeit von 2 Minuten wurde die Keimzahlbestimmung vorgenommen. Nach einer Einwirkzeit von 1 Minute zeigte sich für

| | |
|---|---|
| Pseudomonas aeruginosa eine Reduktion der Keime | auf 2% |
| Candida albicans | auf 0,06% |
| Staphylococcus aureus | auf 0%. |

Aus diesem Ergebnis läßt sich eine sehr gute desinfizierende Wirkung der stabilisierten Wasserstoffperoxid-Lösung ableiten. Weitere Ergebnisse können aus der Tabelle 2 entnommen werden.

## Tabelle 2:

Untersuchungen zur Hemmwirkung in einer Schaumformulierung
Wasserstoffperoxid-Lösung, 2 % (HUWA-SAN)

Testkeim: Pseudomonas aeruginosa, $1 \times 10^9$ KBE/ml

| | Einwirkzeit (Minuten) | | | |
|---|---|---|---|---|
| Testkörper | 0,5 | 1 | 1,5 | 3 |
| I | $2,5 \times 10^5$ | $4,5 \times 10^4$ | $1,5 \times 10^4$ | 0 |
| II | $3 \times 10^5$ | $5 \times 10^4$ | $2 \times 10^4$ | 0 |
| III | $3 \times 10^5$ | $4 \times 10^4$ | $1,5 \times 10^4$ | 0 |
| Kontrolle | $2,9 \times 10^6$ | $2,2 \times 10^6$ | $2,2 \times 10^6$ | $2,1 \times 10^6$ |
| Reduktion auf ca. | 10% | 2% | 8% | 0 |

Testkeim: Candida albicans, $5,5 \times 10^8$ KBE/ml

| | Einwirkzeit (Minuten) | | | |
|---|---|---|---|---|
| Testkörper | 0,5 | 1 | 1,5 | 3 |
| I | $3 \times 10^3$ | $1 \times 10^3$ | $6 \times 10^2$ | $1 \times 10^1$ |
| II | $3,5 \times 10^3$ | $2 \times 10^3$ | $8 \times 10^2$ | 0 |
| III | $5 \times 10^3$ | $9 \times 10^2$ | $8,5 \times 10^2$ | 0 |
| Kontrolle | $1,9 \times 10^6$ | $2 \times 10^6$ | $7 \times 10^5$ | $1 \times 10^5$ |
| Reduktion auf ca. | 0,2% | 0,06% | 0,1% | 0,003% |

Testkeim: Staphylococcus aureus, $2 \times 10^9$ KBE/ml

| | Einwirkzeit (Minuten) | | | |
|---|---|---|---|---|
| Testkörper | 0,5 | 1 | 1,5 | 3 |
| I | $4 \times 10^1$ | 0 | $3 \times 10^1$ | 0 |
| II | $2 \times 10^1$ | 0 | 0 | 0 |
| III | $3,5 \times 10^1$ | 0 | $1 \times 10^1$ | 0 |
| Kontrolle | $3,5 \times 10^6$ | $3,7 \times 10^6$ | $3,7 \times 10^6$ | $3,3 \times 10^6$ |
| Reduktion auf ca. | 0,0009% | 0 | 0,0003% | 0 |

Die weiteren Beispiele zeigen bevorzugte Ausführungsformen der Erfindung:

BEISPIEL 1

Mundwasserzubereitung

Es werden in herkömmlicher Weise gemischt
7 Gew.-% Ethanol,
17 Gew.-% Sorbitol Lösung,
2 Gew.-% stabilisierte $H_2O_2$-Lösung (HUWA-SAN),
1 Gew.-% übliche Zusatzstoffe,
73 Gew.-% Wasser.

BEISPIEL 2

Haftschaum

Es werden gemischt:
1 Gew.-% Polyisosorbat 20,
0,45 Gew.-% Minzöle,
5 Gew.-% Alkohol,
2,20 Gew.-% Natriumbenzoat,
3 Gew.-% Natriumlaurylsulfat,
5 Gew.-% Propan/Butan,
2 Gew.-% stabilisierter Wasserstoffperoxid-Lösung (HUWA-SAN),
1 Gew.-% übliche Zusatzstoffe,
80,35 Gew.-% Wasser.

BEISPIEL 3

Zahnpasta A

12 Gew.-% Diatomeenerde,
1,6 Gew.-% Hydroxyethylcellulose,
12 Gew.-% Glycerin,
2 Gew.-% Siliciumdioxid,
12 Gew.-% Sorbitol-Xylitol-Lösung,
10 Gew.-% Strontiumchlorid,
1 Gew.-% Pfefferminzöl,
2 Gew.-% stabilisierte Wasserstoffperoxid-Lösung (HUWA-SAN),
5 Gew.-% übliche Zusatzstoffe,
42,4 Gew.-% Wasser.

BEISPIEL 4

Zahnpasta B

5 Gew.-% Kaliumnitrat,
1 Gew.-% Natriummonofluorophosphat,
36 Gew.-% Dicalciumphosphat,
12 Gew.-% Sorbitol-Xylitol-Lösung,
12 Gew.-% Glycerin,
1 Gew.-% Pfefferminzöl,
2 Gew.-% stabilisierte Wasserstoffperoxid-Lösung (HUWA-SAN),
5 Gew.-% übliche Zusatzstoffe,
38 Gew.-% Wasser.

**Patentansprüche**

1. Verwendung einer mit einem Silberkolloid stabilisierten Wasserstoffperoxid-Lösung in Mitteln zur Mundhygiene in einer Konzentration von 0,1 bis 10 Gew.-%.

2. Verwendung nach Anspruch 1, dadurch gekennzeichnet, daß die stabilisierte Wasserstoffperoxid-Lösung in einer Konzentration von 1 bis 3 Gew.-% verwendet wird.

3. Verwendung nach Ansprüchen 1 oder 2, dadurch gekennzeichnet, daß die stabilisierte Wasserstoffperoxid-Lösung in Zahnpasta, Zahnpasta für empfindliche Zähne enthaltend lösliche Kalium- und Strontiumsalze oder Hydroxylapatit, Mundwasser, Zahnpulver, Haftmitteln, Haftschaum, Reinigungsmitteln für dritte Zähne, Kaugummi zur Zahnpflege und als Konzentrat oder fertige Lösung zum Befüllen von Mundduschen verwendet wird.

**Claims**

1. The use in mouth-hygiene agents of a hydrogen peroxide solution stabilized by colloidal silver and having a concentration of from 0.1% to 10% by weight.

2. The use according to claim 1, characterized in that said stabilized hydrogen peroxide solution is used in a concentration of from 1% to 3% by weight.

3. The use according to claim 1 or 2, characterized in that said stabilized hydrogen peroxide solution is used in toothpaste, toothpaste for sensitive teeth containing soluble potassium and strontium salts or hydroxylapatite, mouthwash, tooth powder, adhesion agents, adhesion foam, cleaning agents for artificial teeth, chewing gums for dental care, and as a concentrate or instant solution for filling mouth spray devices.

**Revendications**

1. Utilisation, dans des produits pour l'hygiène buccale, d'une solution de peroxyde d'hydrogène stabilisé avec de l'argent colloïdal, à une concentration de 0,1 à 10 % en poids.

2. Utilisation selon la revendication 1, caractérisée en ce que la solution de peroxyde d'hydrogène est utilisée à une concentration de 1 à 3 % en poids.

3. Utilisation selon la revendication 1 ou 2, caractérisée en ce que la solution de peroxyde d'hydrogène stabilisée est utilisée dans une pâte dentifrice, dans une pâte dentifrice pour dents sensibles, contenant des sels solubles de potassium et strontium, ou de l'hydroxyapatite, comme bain de bouche, dans une poudre dentifrice, dans des adhésifs, mousses adhésives et produits de nettoyage pour troisième dentition, dans des gommes à mâcher pour soins dentaires, et comme concentré ou solution prête à l'emploi pour l'exécution de douches buccales.